# EUROPEAN PATENT SPECIFICATION

(11) **EP 1 401 450 B1**
(45) Date of publication and mention of the grant of the patent: **03.01.2007**
(21) Application number: 02743320.0
(22) Date of filing: 04.07.2002
(51) Int. Cl.: A61P 9/04, A61K 31/50

(54) **USE OF A PYRIDAZINONE DERIVATIVE FOR THE TREATMENT OF CONGESTIVE HEART FAILURE**
VERWENDUNG EINES PYRIDAZINON DERIVATES ZUR BEHANDLUNG DES KONGESTIVEN HERZVERSAGENS
UTILISATION D'UN DERIVE DE PYRIDAZINONE POUR LE TRAITEMENT D'UN INFARCTUS CONGESTIF

(30) Priority: 04.07.2001 FI 20011465
(43) Date of publication of application: 31.03.2004
(73) Proprietor: Orion Corporation, 02200 Espoo (FI)
(72) Inventor: HAIKALA, Heimo, FIN-02180 Espoo (FI); SANDELL, Esa-Pekka, FIN-02160 Espoo (FI)
(74) Representative: Sexton, Jane Helen
(86) International application number: PCT/FI2002/000605
(87) International publication number: WO 2003/004035

(56) References cited:
- WO-A-99/16443
- WO-A-99/55305
- WO-A-99/66932
- DATABASE STN INTERNATIONAL [Online] FILE CAPLUS; MYASAKA KATSUHIKO ET AL : "A pyridazinone for treatment of chronic heart failure." retrieved from CAPLUS, accession no. 1993:198216 Database accession no. 118:198216 XP002902781 & JP 04 368328 A (TEIKOKU HORMONE MFG CO. LTD) 21 December 1992 (1992-12-21)

## Description

### Technical field

The invention relates to the use of N-[4-(1,4,5,6-tetrahydro-4-methyl-6-oxo-3-pyridazinyl)phenyl]acetamide (I) for the manufacture of a medicament for reducing mortality in a mammal with congestive heart failure.

### Background of the invention

Congestive heart failure is a major medical problem of growing importance. It is a progressive disease with a poor prognosis. There is ongoing interest in the role of positive inotropic agents (e.g. adenergic agonists or phosphodiesterase III inhibitors) in the treatment of congestive heart failure. However, the enthusiasm for positive inotropic therapy in the congestive heart failure has been dampened by the results of clinical trials, which have shown that these drugs are associated with an increased risk of mortality. Thus, a positive inotropic agent that would have a beneficial effect on the mortality, would be highly desired.

N-[4-(1,4,5,6-tetrahydro-4-methyl-6-oxo-3-pyridazinyl)phenyl]acetamide (I) has been described earlier as a hypotensive agent (US 3,746,712), as a cardiotonic agent having inotropic activity (US 4,397,854) and as a agent for the treatment of chronic heart failure (Japanese Unexamined Patent Publication JP 368328/1992). It has been reported that the inotropic action of (I) is based on phosphodiesterase III inhibition (Ishimori t. et al., Arzneim.- Forsch. (1994), 44(5), 583-8). The compound (I) has an asymmetric carbon atom and may therefore exist in two stereoisomeric forms. The use of the (R)-enantiomer of (I) for the treatment of neurohumoral imbalance caused by alterations of cardiac function to prevent the development of heart failure has been described in WO 99/66932.

Recently is has been found that the (R)-enantiomer of (I) is present as an active metabolite in humans following administration of levosimendan, an agent currently used for the treatment of acute heart failure (see e.g. WO 99/55305).

### Summary of the invention

It has now been unexpectedly discovered that the positive inotropic agent N-[4-(1,4,5,6-tetrahydro-4-methyl-6-oxo-3-pyridazinyl)phenyl]acetamide (I), and particularly its (R)-enantiomer, has an unique ability to reduce mortality in congestive heart failure patients.

The invention provides the use of N-[4-(1,4,5,6-tetrahydro-4-methyl-6-oxo-3-pyridazinyl)phenyl]acetamide (I) or a pharmaceutically acceptable salt thereof in the manufacture of a medicament for reducing mortality of congestive heart failure patients.

The (R)-enantiomer of (I) is particularly preferred agent for reducing mortality of congestive heart failure patients.

### Detailed description of the invention

The method of the invention relates to a administering to a patient with congestive heart failure a mortality reducing amount of N-[4-(1,4,5,6-tetrahydro-4-methyl-6-oxo-3-pyridazinyl)phenyl]acetamide (I) or a pharmaceutically acceptable salt thereof. The (R)-enantiomer of (I) is particularly preferred. The active ingredient of the invention can be prepared using the methods known in the art.

The administration routes of the active ingredient of the invention include, but are not limited to, enteral, e.g. oral or rectal, or parenteral, e.g. intravenous, intramuscular, intraperitoneal or transdermal. In reducing mortality in association with acute heart failure, the active ingredient is preferably administered parenterally, intravenous route being particularly preferred. In reducing mortality in association of chronic heart failure, oral route is particularly preferred.

The daily dose of the active ingredient of the invention in man is generally within the range of 0.05 - 10 mg, depending on the administration route, age, body weight and condition of the patient.

The active ingredient of the invention may be administered e.g. intravenously using an infusion rate, which is from about 0.001 to 1 µg/kg/min, preferably from about 0.005 to 0.5 µg/kg/min. For an intravenous bolus a suitable dose is in the range from about 0.1 to 50 µg/kg, preferably from about 1 to 20 µg/kg. In the treatment of acute heart failure an intravenous bolus followed by continuous infusion may be needed.

The active ingredient of the invention may be administered orally to man in daily dose ranging from about 0.1 to 10 mg, preferably from 0.2 to 5 mg, given once a day or divided into several doses a day, depending on the age, body weight and condition of the patient.

The active ingredient of the invention may be administered periodically, e.g. weekly or biweekly, or daily or several times a day, depending on the patient's needs. The elimination half-life of the active ingredient of the invention in man is rather long, about 72 h. Therefore, a periodical treatment, e.g. weekly, may be satisfactory.

The active ingredient of the invention is formulated into dosage forms using the principles known in the art. It is given to a patient as such or in combination with suitable pharmaceutical excipients in the form of tablets, dragees, capsules, suppositories, emulsions, suspensions or solutions. The composition according to the invention contains a mortality reducing amount of the active ingredient. The content of the active ingredient in the composition is from about 0.5 to 100 % per weight.

Choosing suitable ingredients for the composition is a routine for those of ordinary skill in the art. It is evident that suitable carriers, solvents, gel forming ingredients, dispersion forming ingredients, antioxidants, colours, sweeteners, wetting compounds, release controlling components and other ingredients normally used in this field of technology may be also used.

Formulations suitable for intravenous administration such as injection or infusion formulation, comprise sterile isotonic solutions of the active ingredient and vehicle, preferably aqueous solutions. Typically an intravenous infusion solution comprises from about 0.01 to 0.1 mg/ml of active ingredient. The pharmaceutical formulation may be also in the form of an intravenous infusion concentrate to be diluted with an aqueous vehicle before use. Such concentrate may comprise as a vehicle a pharmaceutically acceptable organic solvent such as dehydrated ethanol.

For oral administration of the active ingredient in tablet form, suitable carriers and excipients include e.g. lactose, corn starch, magnesium stearate, calcium phosphate and talc. For oral administration in capsule form, useful carriers and excipients include e.g. lactose, corn starch, magnesium stearate and talc. For controlled release oral compositions release controlling components can be used. Typical release controlling components include hydrophilic gel forming polymers such as hydroxypropylmethyl cellulose, hydroxypropyl cellulose, carboxymethyl celluloses, alginic acid or a mixture thereof; vegetable fats and oils including vegetable solid oils such as hydrogenated soybean oil, hardened castor oil or castor seed oil (sold under trade name Cutina HR), cotton seed oil (sold under the trade names Sterotex or Lubritab) or a mixture thereof; fatty acid esters such as triglycerides of saturated fatty acids or their mixtures e.g. glyceryl tristearates, glyceryl tripalmitates, glyceryl trimyristates, glyceryl tribehenates (sold under the trade name Compritol) and glyceryl palmitostearic acid ester.

Tablets can be prepared by mixing the active ingredient with the carriers and excipients and compressing the powdery mixture into tablets. Capsules can be prepared by mixing the active ingredient with the carriers and excipients and placing the powdery mixture in capsules, e.g. hard gelatin capsules. Typically a tablet or a capsule comprises from about 0.1 to 10 mg, more typically 0.2 to 5 mg, of the active ingredient.

Salts of the active ingredient, e.g. with inorganic or organic acids, may be prepared by known methods. Pharmaceutically acceptable salts are useful as active medicaments.

The usefulness of the active ingredient of the invention is demonstrated by the following experiments.

### Example 1. Plasma concentrations of (R)-N-[4-(1,4,5,6-tetrahydro-4-methyl-6-oxo-3-pyridazinyl)phenyl]acetamide following levosimendan administration

Plasma concentrations of levosimendan and its active metabolite (R)-N-[4-(1,4,5,6-tetrahydro-4-methyl-6-oxo-3-pyridazinyl)phenyl]acetamide (OR-1896) following administration of 0.2 µg/kg/min of levosimendan for 24 hours in heart failure patients are shown in Figure 1. It can be seen that plasma concentrations of levosimendan are not detectable one day after the discontinuation of infusion, whereas the plasma concentrations of the active metabolite are detectable for 12 days after the infusion. Figure 1 also shows that the elimination half-life of levosimendan is about 1.3 h whereas the elimination half-life of the active metabolite is as long as about 77 h.

It is known that the favourable hemodynamic effects following intravenous administration of levosimendan are seen considerably longer than levosimendan concentrations are observed in plasma. The hemodynamic effects of the active metabolite as such are similar to those of levosimendan. However, it has also been discovered that only about 30 % of the active metabolite is bound to plasma proteins whereas as much as 98 % of levosimendan is bound to plasma proteins. Therefore, the active free concentration of the active metabolite in plasma is proportionally much higher than that of levosimendan.

### Example 2. Mortality reducing effect of (R)-N-[4-(1,4,5,6-tetrahydro-4-methyl-6-oxo-3-pyridazinyl)phenyl]acetamide

The Kaplan Meier curves for all-cause mortality (0-14 days) in congestive heart failure patients follow intravenous levosimendan administration (0.1- 0.2 µg/kg/min) for 24 hours in dobutamine (conventional medication) controlled studies. It can be seen that the survival curves are clearly diverging throughout the observation period. In conclusion, the prolonged beneficial effect seen in survival following levosimendan administration for 24 hours is due to the active metabolite with long elimination half-life leading to persisting effects for days after cessation of infusion.

## Claims

1. Use of N-[4-(1,4,5,6-tetrahydro-4-methyl-6-oxo-3-pyridazinyl)phenyl]acetamide (I) or a pharmaceutically acceptable salt thereof in the manufacture of a medicament for use in reducing mortality in a mammal with congestive heart failure.

2. Use according to claim 1, wherein the (R)-enantiomer of (I) is used.

## Patentansprüche

1. Verwendung von N-[4-(1,4,5,6-Tetrahydro-4-methyl-6-oxo-3-pyridazinyl)-phenyl]acetamid (I) oder einem pharmazeutisch verträglichen Salz davon zur Herstellung eines Medikaments zur Senkung der Mortalität bei einem Säuger mit kongestiver Herzinsuffizienz.

2. Verwendung nach Anspruch 1, wobei das (R)-Enantiomer von (I) verwendet wird.

## Revendications

1. Utilisation du N-[4-(1,4,5,6-tétrahydro-4-méthyl-6-oxo-3-pyridazinyl)phényle]acétamide (I) ou d'un sel pharmaceutiquement acceptable de celui-ci pour la fabrication d'un médicament pour utilisation dans la réduction de la mortalité chez des mammifères atteints d'une insuffisance cardiaque congestive.

2. Utilisation selon la revendication 1, dans laquelle l'énantiomère-(R) du (I) est utilisé.
